# EUROPEAN PATENT SPECIFICATION

(11) **EP 1 236 453 B1**
(45) Date of publication and mention of the grant of the patent: **23.06.2004**
(21) Application number: 00977391.2
(22) Date of filing: 22.11.2000
(51) Int. Cl.: A61F 6/08

(54) **A CONTRACEPTIVE BAG FOR FEMALES**
EMPFÄNGNISVERHÜTENDE TASCHE ZUM EINSETZEN IN DIE VAGINA
POCHE CONTRACEPTIVE POUR FEMMES

(30) Priority: 23.11.1999 CN 99240418
(43) Date of publication of application: 04.09.2002
(73) Proprietor: Feng, Duxiong, Shangai 200080 (CN)
(72) Inventor: Feng, Duxiong, Shangai 200080 (CN)
(74) Representative: Grosse, Rainer, Dipl.-Ing.
(86) International application number: PCT/CN2000/000482
(87) International publication number: WO 2001/037766

(56) References cited:
- WO-A-89/00145
- CN-A- 1 097 974
- CN-U- 86 204 556
- CN-Y- 2 169 397
- DE-C- 828 141
- US-A- 3 683 904
- US-A- 4 066 075
- US-A- 4 867 176
- DATABASE WPI Section Ch, Week 199721 Derwent Publications Ltd., London, GB; Class D22, AN 1997-226856 XP002219564 -& CN 1 097 974 A (FENG D), 1 February 1995 (1995-02-01)

## Description

### BACKGROUND OF THE INVENTION

### 1. Field of the Invention

The present invention relates to reproductive health, and is more particularly related to a female contraceptive device.

### 2. Discussion of the Background

Because modem society faces the challenge of controlling the spread of sexually transmitted disease and overpopulation, development of effective contraceptive measures are vital to addressing these issues. Accordingly, numerous approaches have emerged: the pill, male condom, and various female contraceptive devices (e.g., IUDs (intrauterine devices), diaphragms, and female condoms).

The traditional pill provides a relatively high level of prevention against pregnancy. However, a number of drawblacks attend such a solution. Most significantly, the pill can produce unwanted side effects, such as hormonal disturbance and migraine headaches. Also. the pill does not assist with the prevention of sexually transmitted diseases. Further, administration of the pill requires the assistance of a physician and, therefore, lacks convenience.

The conventional male condom reduces the transmission of diseases, but is not nearly as successful at preventing conception. The relatively poor success rate stems from the fact that the condoms can rupture or, more often than not, are incorrectly used. In addition, couples are sometimes reluctant to use condoms because the male partner experiences reduced or dulling of sexual sensation.

Consequently, research and development has also focused on female contraceptive devices. One such female contraceptive device is the intrauterine device (IUD), which is inserted into the uterus. As such, an IUD is an inconvenient alternative. in that a physician is required properly insert the device. Significantly, the harmful side effects of IUDs are well known.

Additionally, diaphragms have been used to prevent pregnancy. To be effective, the diaphragm must be fitted for the individual female, thus requiring the help of the physician, who also inserts the device. Therefore, accessibility of this contraceptive device is low; namely, users view doctor visitations has highly inconvenient. In addition to being inconvenient. diaphragms are relatively ineffective against unwanted pregnancies.

Another female contraceptive device is the vaginal cover (or female condom), which covers a large amount of the sensitive areas of the vagina. The vaginal cover is a soft membrane blind tube, in which the open end extends and connects to a side ring. Another ring in attached to the other end. The blind tube seals the entirety of the vaginal cavity to block semen from entering the uterus. However, the artificial membrane covers the sexually sensitive areas of the female. thus negatively affecting sexual stimulation and sexual psychological well-being of the female. The female may feel undue anxiety from the fact that the female condom is visibly noticeable. The device is not easily applied, as the device needs to be led in by bottom ring and clamped to the upper side of vaginal cavity. The sexual response is also diminished for the male partner. As applied, the device is aesthetically displeasing. An example of a female condom is disclosed in the United States Patent US 4.867,176.

Another device is suggested in the China Patent CN 1097974 A, which discloses a contraceptive bag to be inserted into the vaginal canal. While the device gives a high contraceptive effect, the application of such device leaves the desire for a device with an improved stimulation.

Based on the foregoing, there is a clear need for improved approaches to raising contraceptive effectiveness.

There is also a need to prevent and reduce sexually transmitted diseases.

There is also a need to permit case of application of the contraceptive device.

There is also a need to develop a contraceptive device that is readily accessible.

There is a further need to enhance sexual stimulation for both the male and female partners.

Based on the need to improve effectiveness of the female contraceptive device, an approach for preventing pregnancies and diseases while promoting sexual stimulation is highly desirable.

### SUMMARY OF THE INVENTION

According to one aspect of the invention, a contraceptive device comprises an upper portion for providing cervical contact. An outer wall has surface protrusions. A lower portion has a plurality of corrugated inner walls that form a plurality of hollow petal-shaped sections with the outer wall. Each of the hollow petal-shaped sections has a curled edge and stores fluid: the hollow petal-shaped sections form a cavity. Under this approach, a female can easily apply the contraceptive device without the aid of a physician.

According to another aspect of the invention, an contraceptive device having a bulbous shape comprises a plurality of petal-shaped sections. Each of the petal-shaped sections has a corrugated inner side. Each of the petal-shaped sections has a curled edge and stores fluid; the plurality of petal-shaped sections form a cavity. A cervical contact side is opposite to the petal-shaped sections. An outer wall has a patterned surface protrusions, which provide a barrier for unidirectional fluid flow. The above arrangement advantageously provides enhanced sexual stimulation for the female and male, while preventing unwanted pregnancies.

### BRIEF DESCRIPTION OF THE DRAWINGS

A more complete appreciation of the invention and many of the attendant advantages thereof will be readily obtained as the same becomes better understood by reference to the following detailed description when considered in connection with the accompanying drawings, wherein:
FIG. **1** is a semi-sectional drawing of the contraceptive device according to one embodiment of the present invention;
FIG. **2** is a diagram showing the contraceptive device with surface protrusions of double-layer walls and three petal-shaped sections, according to the contraceptive device of FIG. **1**;
FIG. **3** is a diagram showing the vaginal canal during two physiological states;
FIG. **4** is a diagram showing the placement of the contraceptive device within the vaginal canal;
FIG. **5** is a diagram showing the contraceptive device with surface protrusions that follow a scale-and-shell pattern, according to one embodiment of the present invention;
FIG. **6** is a diagram showing the contraceptive device with surface protrusions that follow an arc-tile pattern, according to one embodiment of the present invention;
FIG. **7** is a diagram showing the contraceptive device with surface protrusions that follow wavy ring-shaped pattern. according to one embodiment of the present invention;
FIG. **8** is a diagram showing the contraceptive device with surface protrusions that follow a branching pattern, according to one embodiment of the present invention;
FIG. **9** is a diagram showing the contraceptive device with surface protrusions that follow a diamond-shaped pattern. according to one embodiment of the present invention;
FIG. **10** is a semi-sectional drawing of the contraceptive device with a convex upper portion, according to one embodiment of the present invention;
FIG. **11** is a diagram showing the contraceptive device with surface protrusions of double-layer walls and three petal-shaped sections in the contraceptive device of FIG. **10**; and
FIG. **12** is a drawing of the contraceptive device with four petal-shaped sections, according to one embodiment of the present invention.

### DESCRIPTION OF THE PREFERRED EMBODIMENTS

In the following description, for the purpose of explanation, specific details are set forth in order to provide a thorough understanding of the invention. However, it will be apparent that the invention may be practiced without these specific details.

The present invention provides an effective approach to female contraception by exhibiting a high rate of prevention against pregnancy and disease, while providing increased stimulation for the male and female partners. The contraceptive device has an upper portion that contacts the cervix. A lower portion of the contraceptive device has a corrugated inner wall and an outer wall, which form a plurality of hollow petal-shaped sections. Each of the petal-shaped sections is capable of storing semen. The inner wall of the lower portion forms a cavity that receives the penis, which is stimulated by the corrugation on the inner walL The outer wall of the lower portion has surface protrusions that create a barrier to the flow of semen, yet permits the flow of vaginal secretion. The contraceptive device occupies the lower vaginal canal, leaving the upper canal exposed to enhance sensitivity. The bulbous shape of the contraceptive device provides additional sensual stimulation for the female.

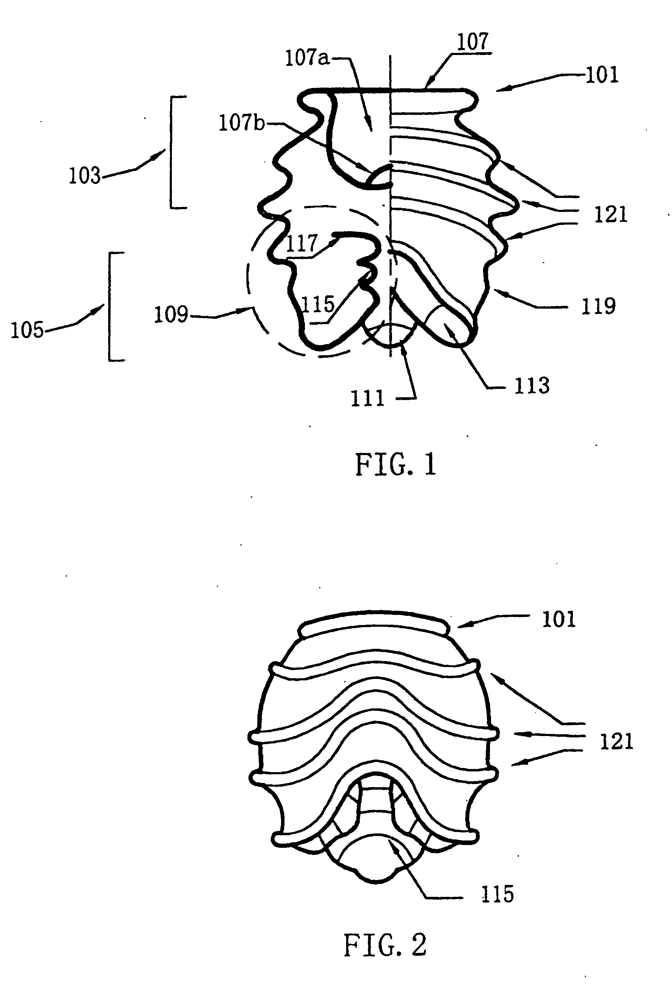

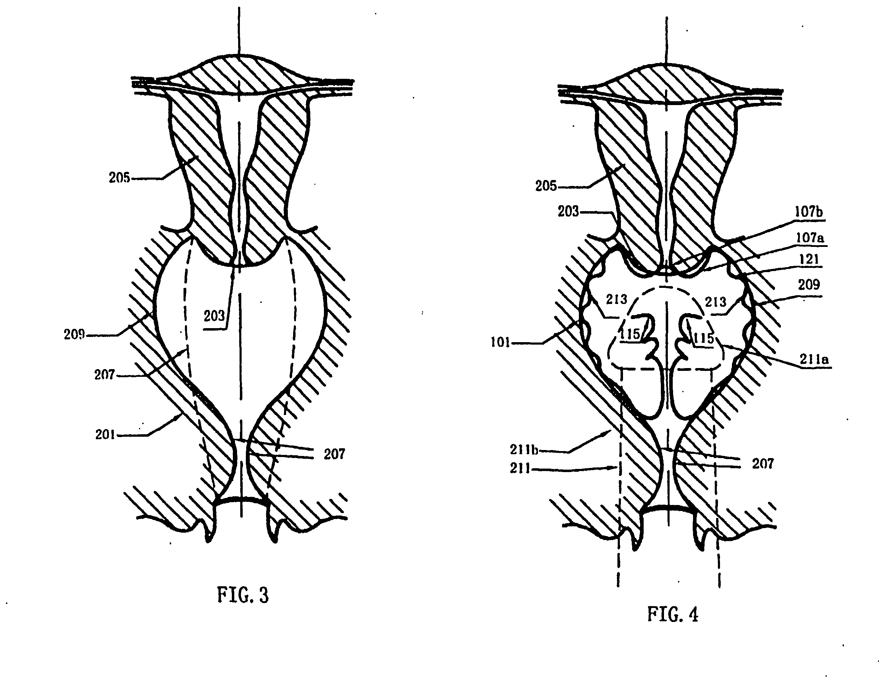

FiG.**1** shows a semi-sectional drawing of the contraceptive device according to one embodiment of the present invention. The female contraceptive device **101,** as shown, has a bulbous shape with an upper portion **103** and a lower portion **105.** The device **101,** according to one embodiment, is made of durable, soft, elastic material, such as latex. In the upper portion **103,** a cervical contact side **107** is concave, forming in a bowl-like cavity **107*a***. At the center of the bowl-like cavity **107*a*** is a reversed hollow navel **107*b***. The lower portion **105** of the device **101** has multiple petal-shaped sections **109, 111,** and **113,** which in this embodiment total three. Each of these petal-shaped sections **109, 111,** and **113** has a corrugated inner wall **115.** According to one embodiment, the corrugation is tripe-like in texture. As will be explained below in FIG. **4,** this corrugated inner wall **115** promotes sexual sensation for the male partner. The petal-shaped sections **109, 111,** and **113** constitute a cavity (or hollow tube) that receives a penis. These petal-shaped sections **109, 111,** and **113** are hollow sacs, which store semen. The semen is prevented from flowing back into the cavity (and ultimately to the vaginal canal), in part, by the curled edge **117** of the inner wall **115.** The curled edge **116** enhances the ability of the device **101** to retain semen, thereby increasing contraceptive effectiveness. In an exemplary embodiment, the tops of the petal-shaped sections **109, 111,** and **113** are cuspidal. The outer wall **119** of the device **101** has surface protrusions **121**, which according to one embodiment form a double-layer wavy ring-shaped pattern. These surface protrusions **121,** as will be more fully discussed with respect to FIG. **3,** control the flow of bodily fluids (e.g., vaginal fluid and semen).

The physical dimensions of the device **101** can vary according to the anatomical characteristics of the female. According to one embodiment, the cervical contact side **107** has a diameter of about 1.50 inches. The height of the device **101** is about 2.25 inches from the cervical contact side **107** to the tip of a petal-shaped section **109**. The middle portion of the device **101** has a diameter of about 2.00 inches.

FIG. **2** shows a side view of the female contraceptive device **101**. As seen in the figure, the surface protrusions **121** curve in a wavy ring-shaped pattern, providing a double-layered outer wall **119**. The corrugated inner wall **115** is also shown. The operation of the device **101** is described below.

FIG. **3** illustrates two states of the vaginal canal. The parts of the female reproductive organs that are directly pertinent to the operation of the female contraceptive device **101** according to various embodiments of the present invention include the vaginal canal **201**, the cervix **203,** and the uterus **205**. Based upon the theory of female anatomy, sexual physiology and psychology, the two states of the vaginal canal 201 involves a non-constricted state and a constricted state. The vaginal canal **201** is essentially is a muscular tube, in which the upper portion, is wider than the lower portion, with the front wall pressing close to the rear wall. As a muscle, the vaginal canal has good extension capacity. Dashed lines **207** denote the non-constricted state; the contraceptive device **101** is inserted during this state.

The female inserts the contraceptive device **101** by folding the device **101** along the vertical axis during the non-constricted state. Because the device **101** is made of a soft material, the device **101** can be rolled into a thin stick and easily inserted; the device **101**, once inserted, automatically decompresses in place. As necessary, lubrication is applied to the contraceptive device **101** before insertion in the vaginal canal **201**. The user inserts until the cervical contact side **107** abuts the cervix **203** (as shown in FIG. **4**). The female can perform this insertion process without the assistance of a physician. During the non-constricted state, the contraceptive device is compressed by the vaginal canal walls.

However, during sexual excitement, the vaginal canal **201** transitions to a constricted state. The narrow lower portion **207**, which constitutes about ¹/₃ of the vaginal canal, constricts, and the upper portion **209** (about ²/₃ of the vaginal canal **201**) fully extends as a result of the uterus **205** raising upward. In the constricted state, the upper portion **209** forms a spherical cavity of about 65 cm in diameter, while the lower portion **207** constricts. Because the contraceptive device **101** is placed in the upper portion **209**, the device **101** expands from its compressed configuration to fulfill the upper portion **209**. The positioning of the device **101** leaves the lower portion **207** of the vaginal canal **201** to exposed. Because the lower portion **207** of vaginal canal **201** is highly sensitive, especially in the front wall (not shown), the female experiences strong sexual stimulation.

The device **101** also provides stimulation for the upper portion **209** of the vaginal canal **201** (i.e., tactile sensation). Although fewer nerves exist in the upper portion **209**, sexual intensity can be greater if properly stimulated. The device **101** provides proper to stimulation by filling the tipper portion **209** of the vaginal canal **201**.

As seen in FIG. **4**, the female contraceptive device **101** rests in the upper portion **209,** whereby the hollow navel **107*b*** is situated at the opening of the cervix **203.** The bowl like cavity **107*a*** surrounds the cervix **203** to anchor the device **101;** that is, this configuration helps secure the device **101** so that the cervical open remains covered. As the female experiences sexual stimulation, vaginal secretion flows from the upper portion **209** of the vaginal canal **201** down to the lower portion **207**. The device **101** does not impede this flow, because the surface protrusions **121** promote fluid progression in the downward direction. That is, the surface protrusions **121** provide a unidirectional flow of fluids from the upper portion **209** of the vaginal canal **201** to the lower portion **207.**

During copulation, the bead **211*a*** of the penis **211** enters the cavity formed by the petal-shaped sections **109, 111,** and **113.** These petal-shaped sections **109**, **111**, and **113** constrict the bead **211*a*** and part of the shaft **211*b***, thereby enhancing the sensation for the male partner. Additionally, the corrugated inner walls **115** supply extra frictional contact to stimulate the bead **211*a***, which is the most sensitive part of the penis **211.** The reversed hollow navel **107*b*** can receive the tip of the bead **211*a***. Upon ejaculation, the semen contacts the bottom side of the hollow navel **107*b***, which diverts the fluid down the sides **213**. The semen is then trapped in the hollow sacs of the petal-shaped sections **109. 111,** and **113.** After completion of sexual activity, the female can easily remove the device **101** by reaching in the vaginal canal **201** and grasping hold of one of the petal-like sections **109, 111,** and **113,** gently guiding the device **101** out the vaginal opening.

The obstruction of sperm from entering the cervix **203** is desired for the purposes of contraception and prevention of venereal diseases. Exposure of the cervix **203** to foreign particles or fluid carrying bacteria is a common source of disease: accordingly, blockage of the cervical opening provides an effective method to disease prevention. For instance, by covering the cervix **203** from semen, the device **101** prevents females effectively from carcinogenesis that can be caused from dirt in the prepuce of the penis **211** entering the cervix **203**. The device **101** achieves successful blockage by fully sealing the cervix **203** and providing an expansion area to fill and seal the spherical cavity of the upper portion **209** of the vaginal canal **201**. The device **101** stores the ejaculated sperm securely within the hollow sacs of the petal-shaped sections **109**, **111**, and **113**. The hollow sacs operate on a similar principle as a one-way valve, as found in the heart, such that the sacs self-close. The surface protrusions **121** are designed to provide essentially unidirectional flow of fluids; therefore, any residual semen not captured within the hollow sacs is impeded from traveling up along the sides of the upper portion **209** of the vaginal canal **201** towards the cervix **203**.

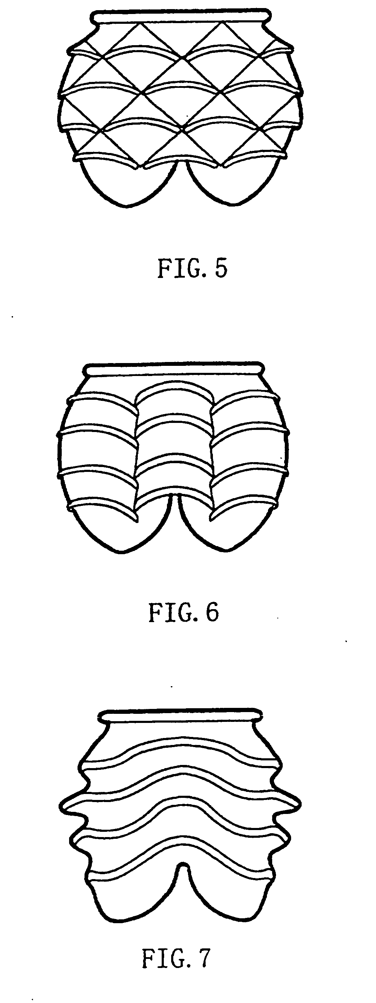

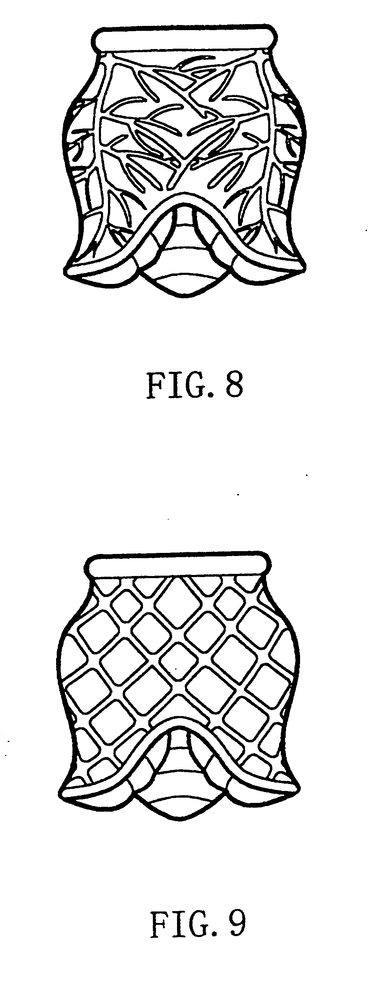

These surface protrusions can follow any number of other patterns (as shown in FIGS. **5-9**). FIG. **5** illustrates surface protrusions **121** in a scale-and-shell pattern. The surface protrusions **121** may also follow an arc-tile pattern, as in FIG. **6.** FIG. **7** shows the device **101** With surface protrusions **121** in a wavy ring-shaped pattern (similar to FIG. **2**). FIG. **8** shows surface protrusions **121** in a branching pattern (or foliage-like). In yet another embodiment, as shown in FIG. **9,** the surface protrusions **121** exhibit a diamond-shaped pattern. The surface protrusions **121,** as mentioned above, direct flow of fluids as well as contribute to the ease of compression of the device **101** for insertion into the vaginal canal **207**.

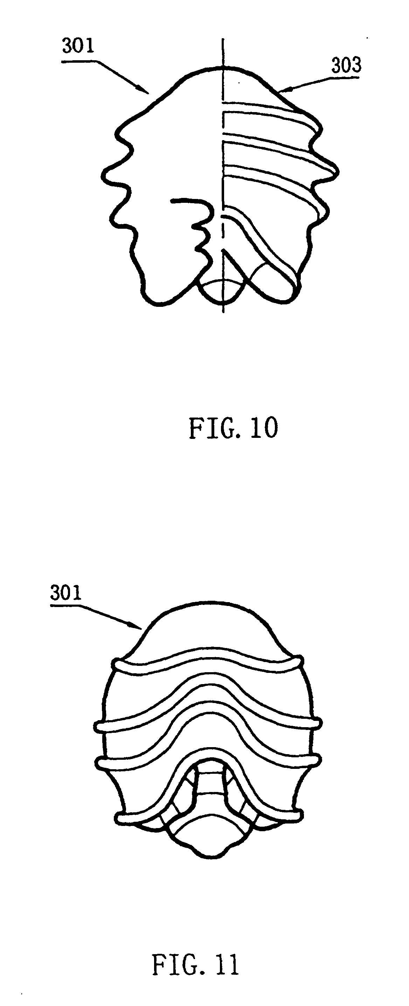

FIGS. **10** and **11** shows an embodiment of the present invention in which the contraceptive device has a convex cervical contact side. The female contraceptive device **301** operates in similar fashion to the contraceptive device of FIGS. **1-2**. The convex cervical contact side **303** reduces manufacturing complexity. Accordingly, the device **301** can be produced more economically that the embodiment of FIG. **1**. Because the device **303** is elastic, the convex cervical contact **303** is dented inwards upon contact with the cervix **203.** In an exemplary embodiment, the device **303** can more readily be produced as a disposable device, with the convenience of the male condom, for instance.

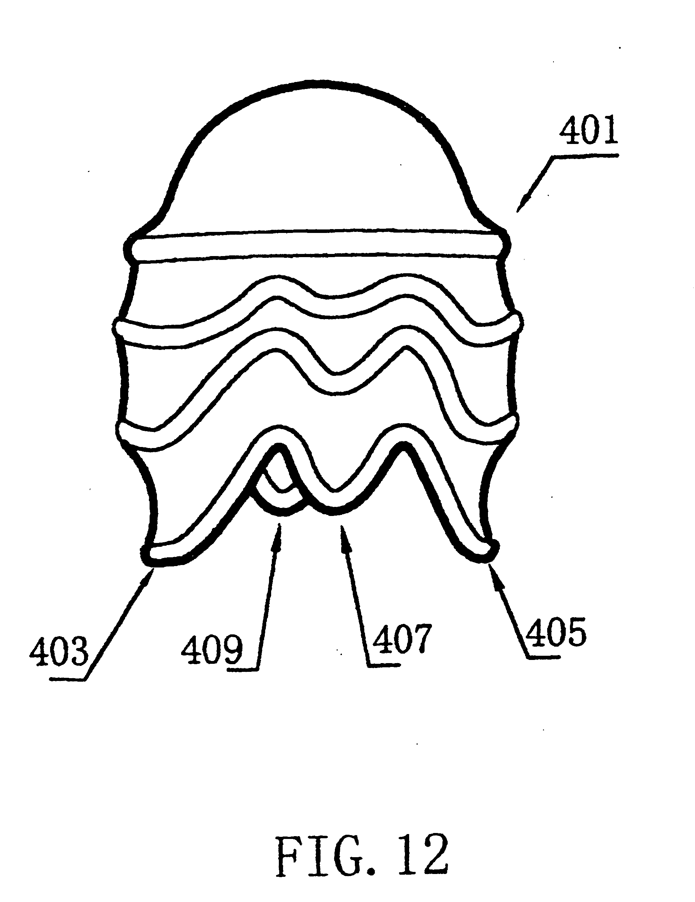

FIG. **12** shows a contraceptive device with a convex cervical contact side and four petal-shaped sections, in accordance with an embodiment of the present invention. Although four petal-shaped sections **403, 405, 407,** and **409** are shown, it is understood that any number of petal-shaped sections can be employed (e.g.. 2). As shown in this figure, the petal-shaped sections **403** and **405,** which are opposite each other, are more cuspidal in shape than the petal-shaped sections **407** and **409**. Additionally, petal-shaped sections **407** and **409** are shorter in length than petal-shaped sections **403** and **405.** The different lengths of the petal-shaped sections provide greater clamping or constriction of the penis **211,** thereby increasing stimulation for the male. The exact design of the device **401** with respect to the number of petals and protrusion surface patterns can be tailored to the individual female. As to the number and shape of petal-shaped sections as well as the surface protrusion patterns, these parameters can be selected depending, on different individuals and their anatomical characteristics. For instance, a greater circumference of the device **401** can be obtained by increasing the number of petal-shaped sections. Manufacturers can thus provide these female contraceptive devices in a variety of configurations, so that the users can experiment for themselves the design best suited to their needs.

The female contraceptive device described herein provides several advantages over prior approaches to contracaption and disease prevention. One advantage is the reduction of sexually transmitted diseases. The device also advantageously provides improved contraceptive effectiveness. Additionally because of the convenience of the contraceptive device, implementation of birth control measures can be rapidly deployed. Importantly, sexual stimulation is enhanced for both the female and male partners, thus increasingly the likelihood that contraception will be used.

Obviously, numerous modifications and variations of the present invention are possible in light of the above teachings. It is therefore to be understood that within the scope of the appended claims, the invention may be practiced otherwise than as specifically described herein.

## Claims

1. A contraceptive device (101) comprising:
an upper portion (103) having a convex side for providing cervical contact;
an outer wall having surface protrusions (121); and
a lower portion (105) having a plurality of corrugated inner walls (115) that form a plurality of hollow petal-shaped sections (109,111,113) with the outer wall each of the hollow petal-shaped sections having a curled edge (117) and being configured to store fluid, the hollow petal-shaped sections forming a cavity,
**characterised by** at least two of the petal-shaped sections being of different lengths.

2. The device according to claim 1, wherein each of the inner walls has tripe-like corrugation.

3. The device according to claim 1, wherein the surface protrusions follow a scale-and-shell pattern.

4. The device according to claim 1, wherein the surface protrusions follow an arc-tile pattern.

5. The device according to claim 1, wherein the surface protrusions follow a wavy ring-shaped pattern.

6. The device according to claim 1, wherein the surface protrusions follow a diamond-shaped pattern.

7. The device according to claim 1, wherein the surface protrusions follow a branching pattern.

8. The device according to one of the previous claims, wherein quantity of the plurality of petal-shaped sections is two, three, or four.

9. The device according to one of the previous claims having a bulbous shape.

10. The device according to one of the previous claims having an outer wall with a patterned surface.

## Patentansprüche

1. Verhütungsmittel (101), umfassend:
einen oberen Teil (103), der eine konvexe Seite zur Herstellung des Kontakts mit der Zervix besitzt;
eine Außenwand, die Vorsprünge (121) besitzt; und
einen unteren Teil (105), der eine Vielzahl geriffelter Innenwände (115) besitzt, die eine Vielzahl hohler, blütenblattförmiger Abschnitte (109, 111, 113) mit der Außenwand bilden, wobei jeder der hohlen, blütenblattförmigen Abschnitte eine geschwungene Begrenzung (117) besitzt und so gestaltet ist, dass er Flüssigkeit speichern kann, indem die hohlen, blütenblattförmigen Abschnitte einen Hohlraum bilden;
**dadurch gekennzeichnet, dass** mindestens zwei der blütenblattförmigen Abschnitte von unterschiedlicher Länge sind.

2. Verhütungsmittel nach Anspruch 1, bei dem jede der Innenwände eine kaldaunenähnliche Riffelung aufweist.

3. Verhütungsmittel nach Anspruch 1, bei dem die Vorsprünge der Oberfläche einer Schuppen- und Muschelstruktur folgen.

4. Verhütungsmittel nach Anspruch 1, bei dem die Vorsprünge der Oberfläche einer Bogen-Ziegel-Struktur folgen.

5. Verhütungsmittel nach Anspruch 1, bei dem die Vorsprünge der Oberfläche einer welligen ringförmigen Struktur folgen.

6. Verhütungsmittel nach Anspruch 1, bei dem die Vorsprünge der Oberfläche einer rautenförmigen Struktur folgen.

7. Verhütungsmittel nach Anspruch 1, bei dem die Vorsprünge der Oberfläche einer verästelten Struktur folgen.

8. Verhütungsmittel nach einem der vorhergehenden Ansprüche, bei dem die Menge der Vielzahl blütenblattförmiger Abschnitte zwei, drei oder vier beträgt.

9. Verhütungsmittel nach einem der vorhergehenden Ansprüche, das eine bauchige Form besitzt.

10. Verhütungsmittel nach einem der vorhergehenden Ansprüche, das eine Außenwand mit einer strukturierten Oberfläche besitzt.

## Revendications

1. Dispositif contraceptif (101) comprenant :
une partie supérieure (103) présentant un côté convexe destiné à fournir un contact cervical,
une paroi extérieure comportant des saillies en surface (121), et
une partie inférieure (105) comportant une pluralité de parois internes plissées (115) qui forment une pluralité de sections en forme de pétales creuses (109, 111, 113), la paroi extérieure de chacune des sections en forme de pétales creuses comportant un bord crispé (117) et étant configurées pour stocker un fluide, les sections en forme de pétales creuses formant une cavité,
**caractérisé par** au moins deux des sections en forme de pétales qui sont de longueurs différentes.

2. Dispositif selon la revendication 1, dans lequel chacune des parois internes présente une ondulation à la manière d'un intestin.

3. Dispositif selon la revendication 1, dans lequel les saillies en surface suivent un motif en forme de coquilles en série ou en écailles.

4. Dispositif selon la revendication 1, dans lequel les saillies en surface suivent un motif de tuiles en arc.

5. Dispositif selon la revendication 1, dans lequel les saillies en surface suivent un motif en forme d'anneau ondulé.

6. Dispositif selon la revendication 1, dans lequel les saillies en surface suivent un motif en forme de diamant.

7. Dispositif selon la revendication 1, dans lequel les saillies en surface suivent un motif de ramifications.

8. Dispositif selon l'une des revendications précédentes, dans lequel la quantité de la pluralité des sections en forme de pétales est de deux, trois ou quatre.

9. Dispositif selon l'une des revendications précédentes présentant une forme de bulbe.

10. Dispositif selon l'une des revendications précédentes comportant une paroi externe présentant une surface à motifs.
